(19) Europäisches Patentamt
European Patent Office
Office européen des brevets

(11) EP 0 709 384 B1

(12) # EUROPÄISCHE PATENTSCHRIFT

(45) Veröffentlichungstag und Bekanntmachung des Hinweises auf die Patenterteilung:
**23.12.1998 Patentblatt 1998/52**

(51) Int Cl.6: **C07D 401/06**, C07D 413/06, C07D 417/06, A61K 31/445, A61K 31/55

(21) Anmeldenummer: **95116458.1**

(22) Anmeldetag: **19.10.1995**

(54) **Benzylpiperidinderivate mit hoher Affinität zu Bindungsstellen von Aminosäure-Rezeptoren**

Benzylpiperidine derivatives having high affinity for binding sites of aminoacid receptors

Dérivés de benzylpiperidine ayant une haute affinité pour les sites de liaison de récepteurs d'aminoacides

(84) Benannte Vertragsstaaten:
**AT BE CH DE DK ES FR GB GR IE IT LI LU NL PT SE**
Benannte Erstreckungsstaaten:
**LT SI**

(30) Priorität: **31.10.1994 DE 4438810**
**19.07.1995 DE 19526269**

(43) Veröffentlichungstag der Anmeldung:
**01.05.1996 Patentblatt 1996/18**

(73) Patentinhaber: **MERCK PATENT GmbH**
**64293 Darmstadt (DE)**

(72) Erfinder:
- **Prücher, Helmut**
**D-64646 Heppenheim (DE)**
- **Leibrock, Joachim, Dr.**
**D-64347 Griesheim (DE)**
- **Gottschlich, Rudolf, Dr.**
**D-64354 Reinheim (DE)**
- **Schwartz, Harry, Dr.**
**D-65719 Hofheim-Diedenbergen (DE)**

(56) Entgegenhaltungen:
**EP-A- 0 351 282**
**FR-A- 2 688 504**
**US-A- 4 455 422**

- **PATENT ABSTRACTS OF JAPAN vol. 006 no. 239 (C-137) ,26.November 1982 & JP-A-57 142972 (OTSUKA SEIYAKU KK) 3.September 1982,**
- **CHEMICAL ABSTRACTS, vol. 118, no. 23, 1993 Columbus, Ohio, US; abstract no. 233858f, CHENARD ET AL. 'Oxindole N-methyl-D-aspartate (NMDA) antagonists'**

Bemerkungen:
Die Akte enthält technische Angaben, die nach dem Eingang der Anmeldung eingereicht wurden und die nicht in dieser Patentschrift enthalten sind.

EP 0 709 384 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Benzylpiperidinderivate der Formel I

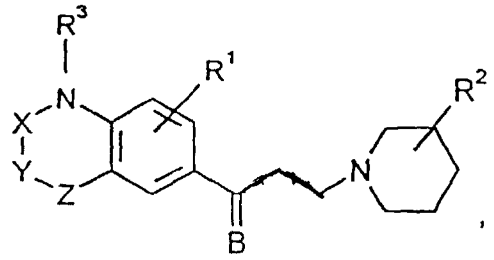

(I)

worin

$R^1$ H, Hal oder Nitro,

$R^2$ eine unsubstituierte oder durch Hal am Aromaten substituierte Benzylgruppe in 2-, 3- oder 4-Stellung des Piperidinrings, mit der Maßgabe, daß $R^2 \neq$ 4-Benzyl, wenn X -CO-, Y und Z $-CH_2-$ und $R^1$ H bedeuten,

$R^3$ H oder A,

X -CO- oder $-SO_2-$,

Y $-CH_2-$, -NH-, -O-, -S- oder auch -CO-, wenn X -CO- und Z -NH-, -NA-,

Z $-CH_2-$, $-C(A)_2-$, $-CH_2CH_2-$, -CH=CH-, -CO-, -NH-, -NA-, -O-, oder eine Bindung,

wobei einer der Reste X, Y und Z -O-, -S- oder -NH- sein kann, jedoch X-Y bzw. Y-Z nicht -0-0-, -S-S-, -NH-O-, -O-NH-, -NH-NH-, -O-S-, -S-O- ist,

A Alkyl mit 1-6 C-Atomen und

B O, H + OH,

Hal F, Cl, Br oder I

bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze wertvolle pharmakologische Eigenschaften besitzen. Sie zeigen eine hohe Affinität zu Bindungsstellen von Aminosäure-Rezeptoren, insbesondere zur Glycin- Polyamin- und/oder NMDA-Bindungsstelle des NMDA-Rezeptors (NMDA = N-Methyl-D-aspartat). Die Verbindungen eignen sich daher zur Behandlung von neurodegenerativen Erkrankungen einschließlich cerebrovaskularen Krankheiten. Ebenso können die neuen Wirkstoffe als Analgetika oder Anxiolytika sowie zur Behandlung von Epilepsie, Schizophrenie, der Alzheimer-, der Parkinson-bzw. Huntington-Krankheit, cerebralen Ischämien oder Infarkten verwendet werden. Ferner eignen sie sich zur Behandlung von Psychosen, bedingt durch überhöhte Aminosäurespiegel.

Der [$^3$H]-CGP-39653-Bindungstest für die Glutamat-Bindungsstelle des NMDA-Rezeptors kann beispielsweise nach der Methode von M.A. Stills et al., beschrieben in Eur. J. Pharmacol. 192, 19-24 (1991), durchgeführt werden. Der Test für die Glycin-Bindungsstelle des NMDA-Rezeptors ist durchführbar nach der Methode von M.B. Baron et al., beschrieben in Eur. J. Pharmacol. 206, 149-154 (1991). Die in-vitro-Aminosäurefreisetzung ist gemäß der Methode von D. Lobner und P. Lipton (Neurosci. Lett. 117, 169-174 (1990)) nachweisbar.

Die Wirkung gegen Morbus Parkinson, d.h. die Potenzierung des L-DOPA-induzierten kontralateralen Drehens bei hemiparkinsonischen Ratten, ist nach der Methode von U. Ungerstedt und G.W. Arbuthnott, Brain Res 24, 485 (1970) nachweisbar.

Besonders geeignet sind die Verbindungen zur Behandlung oder Prophylaxe von Schlaganfällen sowie zum Schutz vor und zur Behandlung von Hirnödemen und Unterversorgungszuständen des Zentralnervensystems, vor allem Hypoxie oder Anoxie.

Die genannten Wirkungen können außerdem nach den Methoden nachgewiesen oder überprüft werden, wie sie in den folgenden Literaturstellen beschrieben sind:

J.W. McDonald, F.S. Silverstein und M.v. Johnston, Eur. J. Pharmacol. 140, 359 (1987); R. Gill, A.C. Foster und G.N. Woodruff, J. Neurosci. 7, 3343 (1987); S.M. Rothmann, J.H. Thurston, R.E. Hauhart, G.D. Clark und J.S. Soloman, Neurosci. 21, 73 (1987) oder M.P. Goldbert, P.-C. Pham und D.W. Choi, Neurosci. Lett. 80, 11 (1987).

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Die JP-A-57 142972 und die US-A-4 455 422 beschreiben Carbostyril-Derivate und deren Salze, die eine Antihistamin-Wirkung besitzen sowie eine Inhibitonvirkung auf das ZNS ausüben.

Die EP-A- 0 351 282 beschreibt Derivate von (1-Hydroxy 2-piperidinyl alkyl) indolon-2, Quinoleinon-2, Benzoazepidon-2, Benzimidazolon-2 und Quinazolinon-2 deren Herstellung sowie Verwendung als Therapeutika.

Die FR-A-2 688 504 beschreibt Derivate von 2-(piperidin-1-yl) ethanol deren Herstellung sowie Verwendung als Therapeutika.

Das Abstract C.A. 118: 233858 beschreibt Oxindol-N-methyl-D-aspartat (NMDA)-Antagonisten deren Herstellung sowie Verwendung als Therapeutika.

Gegenstand der Erfindung sind Verbindungen der Formel I sowie ihre Salze.

Die Gruppe A steht für Alkyl mit 1, 2, 3, 4, 5 oder 6 C-Atomen, insbesondere für Methyl oder Ethyl, aber auch für Propyl, Isopropyl, Butyl, Isobutyl, sek.-Butyl, tert.-Butyl.

Die Gruppe Hal ist vorzugsweise Cl, weiterhin bevorzugt F.

Der Rest $R^1$ ist vorzugsweise H oder Cl.

Der Rest $R^2$ ist vorzugsweise unsubstituiertes Benzyl, ferner bevorzugt 2-, 3- oder 4-Fluorbenzyl, weiterhin 2-, 3- oder 4-Chlorbenzyl, 2-, 3- oder 4-Brombenzyl, 2-, 3- oder 4-Iodbenzyl.

Der Rest X ist vorzugsweise -CO-.

Der Rest Y ist vorzugsweise -NH-, ferner bevorzugt $-CH_2-$ oder -O-.

Der Rest Z ist vorzugsweise eine Bindung, ferner bevorzugt $-CH_2-$, $C(CH_3)_2-$ oder -O-.

Dementsprechend ist die Gruppe -X-Y-Z- bevorzugt -CO-NH-, ferner bevorzugt $-CO-CH_2-CH_2-$, $-CO-CH_2-C(CH_3)_2-$, $-CO-CH_2-O-$, $-CO-O-CH_2-$, $-CO-NH-CH_2-$ oder -CO-O- sowie auch -CO-S- oder $CO-CH_2-$.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können auch durch die nachstehenden Formeln Ia bis Ih ausgedrückt werden, die der Formel I entsprechen und worin die nicht näher bezeichneten Reste die bei der Formel I angegebene Bedeutung haben, worin jedoch

in Ia $R^1$ H bedeutet;

in Ib $R^1$ H oder Cl bedeutet;

in Ic -X-Y-Z- -CO-NH-, $-CO-CH_2-CH_2-$, $-CO-CH_2-C(CH_3)_2-$, $-CO-CH_2-O-$, $-CO-O-CH_2-$, $-CO-NH-CH_2-$ oder -CO-O- sowie auch -CO-S- oder $-CO-CH_2-$ bedeutet;

in Id -X-Y-Z- -CO-NH-, $-CO-CH_2-CH_2-$ oder $-CO-CH_2-C(CH_3)_2-$ bedeutet;

in Ie -X-Y-Z- -CO-NH- oder $-CO-CH_2-CH_2-$ bedeutet;

in If $R^1$ H oder Cl und -X-Y-Z- -CO-NH-, $-CO-CH_2-CH_2-$ oder $-CO-CH_2-C(CH_3)_2-$ bedeuten;

in Ig $R^1$ H oder Cl und -X-Y-Z- -CO-NH- oder $-CO-CH_2-CH_2-$ bedeuten;

in Ih $R^1$ H und -X-Y-Z- -CO-NH- bedeuten.

Weiterhin sind bevorzugt Verbindungen der Formeln I' sowie Ia' bis Ih', die den Formeln I bzw. Ia bis Ih entsprechen, worin jedoch $R^2$ eine unsubstituierte Benzylgruppe bedeutet.

Gegenstand der Erfindung ist ferner ein Verfahren zur Herstellung von Benzylpiperidinderivaten der oben angegebenen Formel I sowie von deren Salzen, dadurch gekennzeichnet, daß man eine sonst der Formel I entsprechende Verbindung, die jedoch an Stelle eines oder mehrerer H-Atome eine oder mehrere reduzierbare Gruppen und/oder

eine oder mehrere zusätzliche Bindungen enthält, mit einem reduzierenden Mittel behandelt, oder daß man eine Verbindung der Formel II

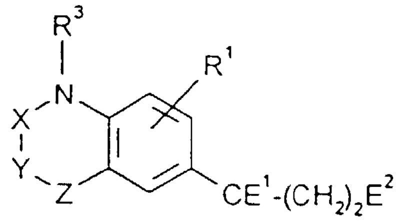

(II)

worin

| | |
|---|---|
| $E^1$ | O oder H + OH, |
| $E^2$ | Cl, Br, J oder eine reaktionsfähige veresterte OH-Gruppe bedeuten oder |
| $E^1$ und $E^2$ | zusammen auch ein O-Atom bedeuten können und |
| $R^1$, $R^3$, X, Y, Z und Z | die angegebenen Bedeutungen haben, |

mit einer Verbindung der Formel III

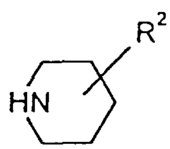

(III)

worin

$R^2$ die angegebene Bedeutung hat,

umsetzt,
und/oder daß man eine erhaltene Base der Formel I durch Behandeln mit einer Säure in eines ihrer Säureadditionssalze umwandelt.

Die Verbindungen der Formel I werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart oder in J. March, Adv. Org. Chem., 3rd Ed., J. Wiley & Sons (1985)) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man sie aus dem Reaktionsgemisch nicht isoliert, sondern sofort weiter zu den Verbindungen der Formel I umsetzt.

Andererseits ist es möglich, die Reaktion stufenweise durchzuführen, wobei man weitere Zwischenprodukte isolieren kann.

Die einzelnen Verfahrensvarianten werden im folgenden näher erläutert.

Verbindungen der Formel I, in denen B H + OH bedeutet, werden bevorzugt hergestellt durch Reduktion entsprechender Vorprodukte, die reduzierbare Gruppen und/oder zusätzliche Bindungen enthalten. Bevorzugt werden sie aus den entsprechenden Ketonen der Formel IV

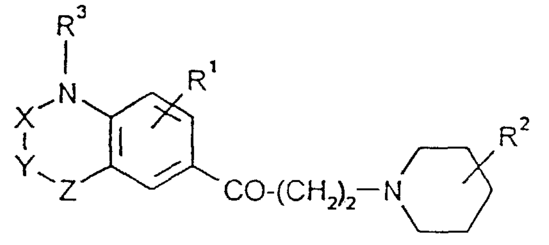

(IV)

worin

$R^1$, $R^2$, $R^3$, X, Y, und Z die bei Formel I angegebenen Bedeutungen haben, hergestellt.

Sie und die durch die Formel I definierten Ketone sind z.B. erhältlich durch Friedel-Crafts-Acylierung der in der Regel bekannten Verbindungen der Formel V

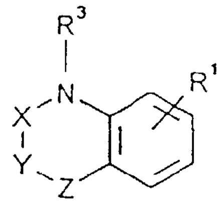

(V)

mit entsprechenden endständig chlorierten Alkylcarbonsäurechloriden, insbesondere mit Chloracetylchlorid, 1-Chlorpropionylchlorid oder mit 1-Chlorbutyrylchlorid, und anschließende Reaktion mit einem 2-, 3- oder 4-$R^2$-piperidin der Formel III.

Als Reduktionsmittel eignen sich vorzugsweise katalytisch erregter oder nascierender Wasserstoff sowie komplexe Metallhydride, z.B. Alkalimetall-aluminium- oder Alkalimetall-bor-hydride.

Als Katalysatoren bei katalytischen Hydrierungen kommen bevorzugt Edelmetallkatalysatoren wie Platin oder Palladium in Betracht, die auf einem Träger wie Kohle vorliegen können, ferner Raney-Metalle wie Raney-Nickel oder Kupfer-Chrom-Oxid. Hydrierungen werden zweckmäßig bei Drucken zwischen 0 und 200 bar und bei Temperaturen zwischen 0 und 150°, vor allem zwischen 15 und 100° ausgeführt.

Als Lösungsmittel eignen sich z.B. Alkohole wie Methanol, Ethanol oder Isopropanol, Ether wie Tetrahydrofuran (THF) oder Methyl-tert.-butylether, Ester wie Ethylacetat, Amide wie Dimethylformamid (DMF), Sulfoxide wie Dimethylsulfoxid (DMSO).

Bevorzugt ist die Reduktion der Ketone IV mit einem komplexen Metallhydrid, insbesondere $NaBH_4$. Diese wird zweckmäßig in einem Alkohol wie Methanol bei Temperaturen zwischen 0 und 30°, vorzugsweise 5 und 15° vorgenommen. Bei schwer löslichen Ausgangsstoffen empfiehlt sich ein Zusatz eines weiteren Lösungsmittels wie THF. Das Reduktionsmittel wird zweckmäßig in großem Überschuß eingesetzt, z.B. im Molverhältnis 1:1.

Bei der Reduktion der Ketone IV entsteht in der Regel ein Gemisch der beiden epimeren Hydroxyverbindungen. Verwendet man ein chirales Reduktionsmittel, z.B. (+)- oder (-)-β-Chlor-diisopinocamphenyl-boran, so kann man auch bevorzugt oder ausschließlich eines der Epimeren erhalten. Das gleiche gelingt durch Reduktionen mit dazu geeigneten Mikroorganismen, insbesondere solchen der Gattungen Candida oder Rhodutorula, z.B. Rhodutorula mucilaginosa.

Verbindungen der Formel I, in denen B H + OH bedeutet, sind ferner erhältlich durch Reaktion von Halohydrinen oder Epoxiden der Formel II mit 2-, 3- oder 4-$R^2$-Piperidinen der Formel III.

Die Ausgangsstoffe der Formel II sind beispielsweise erhältlich durch die genannte Friedel-Crafts-Acylierung von Verbindungen der Formel V und gegebenenfalls anschließende Reduktion sowie, falls erwünscht, HCl-Abspaltung unter Epoxid-Bildung.

Verbindungen der Formel III sind in der Regel bekannt und im Handel erhältlich.

Die Umsetzung von II mit III gelingt zweckmäßig in Anwesenheit oder Abwesenheit eines der genannten Losungsmittel in Gegenwart oder Abwesenheit eines Kondensationsmittels, z.B. einer Base, bei Temperaturen zwischen -20 und 200°, vorzugsweise 0 und 100°. Als Basen eignen sich z.B. Alkalimetallhydroxide wie NaOH oder KOH, Alkalimetallcarbonate wie $Na_2CO_3$ oder $K_2CO_3$, tertiäre Amine wie Triethylamin oder Pyridin. Als Lösungsmittel ist Ethanol, als Base Triethylamin besonders bevorzugt.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder

Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I besitzen mindestens zwei Asymmetriezentren, wenn $R^2$ = 2- oder 3-Benzyl und B = H + OH ist. Sie können daher bei ihrer Herstellung als Gemisch von Racematen oder, falls optisch aktive Ausgangsstoffe verwendet werden, auch in optisch aktiver Form erhalten werden. Aus den Racematgemischen kann man die einzelnen Racemate, beispielsweise durch Umkristallisieren aus inerten Lösungsmitteln, in reiner Form isolieren. Erhaltene Racemate können, falls erwünscht, nach an sich bekannten Methoden mechanisch oder chemisch in ihre Enantiomeren getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/ oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Schmerzzuständen, aber auch zur Minderung der Folgeschäden nach einer Ischämie verwendet werden. Besonders eignen sich die Verbindungen zur Behandlung von neurodegenerativen Erkrankungen bzw. von Erkrankungen, die durch eine Fehlfunktion an der Glycin- Polyamin- oder Glutamat-Bindungsstelle des NMDA-Rezeptors hervorgerufen werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. Prozentangaben bedeuten Gewichtsprozente. Die Bezeichnungen "S" und "R" beziehen sich auf das chirale C-Atom im Piperidinring, sofern nichts anderes angegeben ist.

In den nachfolgenden Beispielen bedeutet "übliche Aufarbeitung": Man gibt, falls erforderlich, Wasser oder verdünnte Natronlauge hinzu, extrahiert mit Dichlormethan, trennt ab, trocknet die organische Phase mit Natriumsulfat,

filtriert, dampft ein und reinigt durch Chromatographie an Kieselgel und/oder durch Kristallisation. [α] = $[\alpha]_D^{20}$, c = 1 in DMSO.

**Beispiel 1**

Eine Lösung von 36,2 g 1,2,3,4-Tetrahydro-6-(2-(3-benzyl-piperidino)-1-oxo-ethyl)-chinolin-2-on ("A"; Racemat; erhältlich durch Reaktion von 1,2,3,4-Tetrahydro-chinolin-2-on mit Chloracetylchlorid/$AlCl_3$/DMF zu 6-Chloracetyl-1,2,3,4-tetrahydro-chinolin-2-on ("B") und anschließende Umsetzung mit (racemischem) 3-Benzyl-piperidin in Ethanol in Gegenwart von Triethylamin) in 725 ml Methanol wird mit 3,8 g $NaBH_4$ versetzt und dann 2 Std. bei 10° gerührt. Man arbeitet wie üblich auf (Natronlauge/Dichlormethan) und erhält 6-(2-(3-Benzyl-piperidino)-1-hydroxy-ethyl)-1,2,3,4-tetrahydro-chinolin-2-on, Racemat ("C").

Analog erhält man mit $NaBH_4$:

aus (S)-"A" (F. 155-157°; [α] +18,8°; erhältlich aus "B" und 3S-Benzyl-piperidin):
(S)-"C"; Hydrochlorid, F. 209-211°; [α] +30,4°;

aus (R)-"A" (erhältlich aus "B" und 3R-Benzyl-piperidin):
(R)-"C"; F. 136-138°; Hydrochlorid, F. 209-211°; [α] -37,5°;

aus 1,2,3,4-Tetrahydro-6-(2-(2-benzyl-piperidino)-1-oxo-ethyl)-chinolin-2-on ("D"; Racemat; erhältlich durch Reaktion von "B" mit 2-Benzyl-piperidin):
6-(2-(2-Benzyl-piperidino)-1-hydroxy-ethyl)-1,2,3,4-tetrahydro-chinolin-2-on, Racemat ("E");

aus (S*)-"D" (erhältlich aus "B" und 2S*-Benzyl-piperidin):
(S*)-"E", Harz;

aus (R*)-"D" (erhältlich aus "B" und 2R*-Benzyl-piperidin):
(R*)-"E", Harz;

aus 7-(2-(3-Benzyl-piperidino)-1-oxo-ethyl)-2,3-dihydro-1,4-benzoxazin-3-on ("F"; erhältlich durch Reaktion von 2,3-Dihydro-1,4-benzoxazin-3-on mit Chloracetylchlorid zu 7-Chloracetyl-2,3-dihydro-4H-1,4-benzoxazin-3-on ("G"), dann Umsetzung mit 3-Benzyl-piperidin):
7-(2-(3-Benzylpiperidino)-1-hydroxy-ethyl)-2,3-dihydro-4H-1,4-benzoxazin-3-on, Racemat ("H");

aus (S)-"F" (erhältlich aus "G" und 3S-Benzyl-piperidin):
(S)-"H"; Hydrochlorid, amorph, Zers. bei 130°; [α] +25,0°;

aus (R)-"F" (erhältlich aus "G" und 3R-Benzyl-piperidin):
(R)-"H", Hydrochlorid, amorph, Zers. bei 117°; [α] -24,5°;

aus 7-(2-(2-Benzyl-piperidino)-1-oxo-ethyl)-2,3-dihydro-4H-1,4-benzoxazin-3-on ("I"; Racemat; erhältlich durch Reaktion von "G" mit 2-Benzyl-piperidin):
7-(2-(2-Benzyl-piperidino)-1-hydroxy-ethyl)-2,3-dihydro-4H-1,4-benzoxazin-3-on, Racemat ("J");

aus (S*)-"I" (erhältlich aus "G" und 2S*-Benzyl-piperidin):
(S*)-"J", F. 132-135°; [α] -26,0°;

aus (R*)-"I" (erhältlich aus "G" und 2R*-Benzyl-piperidin):
(R*)-"J", F. 135° (Zers.); [α] +27,0°;

aus 5-(2-(3-Benzyl-piperidino)-1-oxo-ethyl)-2,3-dihydro-benzimidazol-2-on ("K"; erhältlich durch Reaktion von 2,3-Dihydro-benzimidazol-2-on mit Chloracetylchlorid zu 5-Chloracetyl-2,3-dihydro-benzimidazol-2-on ("L"), dann Umsetzung mit 3-Benzyl-piperidin):
5-(2-(3-Benzyl-piperidino)-1-hydroxy-ethyl)-2,3-dihydro-benzimidazol-2-on, Racemat ("M");

aus (S)-"K" (erhältlich aus "L" und 3S-Benzyl-piperidin):
(S)-"M"; F. 164-167°; [α] +30,7°;

aus (R)-"K" (erhältlich aus "L" und 3R-Benzyl-piperidin):
(R)-"M", F. 163-166°; [α] -31,7°;

aus 5-(2-(2-Benzyl-piperidino)-1-oxo-ethyl)-2,3-dihydro-benzimidazol-2-on ("N"; erhältlich durch Reaktion von "L" mit 2-Benzyl-piperidin):
5-(2-(2-Benzyl-piperidino)-1-hydroxy-ethyl)-2,3-dihydro-benzimidazol-2-on, Racemat ("O");

aus (S*)-"N" (erhältlich aus "L" und 2S*-Benzyl-piperidin):
(S*)-"O";

aus (R*)-"N" (erhältlich aus "L" und 2R*-Benzyl-piperidin):
(R*)-"O";

aus 6-(2-(3-Benzyl-piperidino)-1-oxo-ethyl)-2,3-dihydro-benzoxazol-2-on ("P"; erhältlich durch Reaktion von 2,3-Dihydro-benzoxazol-2-on mit Chloracetylchlorid zu 6-Chloracetyl-2,3-dihydro-benzoxazol-2-on ("Q"), dann Umsetzung mit 3-Benzyl-piperidin):
6-(2-(3-Benzyl-piperidino)-1-hydroxy-ethyl)-2,3-dihydro-benzoxazol-2-on, Racemat ("R");

aus (S)-"P" (erhältlich aus "Q" und 3S-Benzyl-piperidin):
(S)-"R", amorph, Zers. bei 159°; [α] +23,8°;

aus (R)-"P" (erhältlich aus "Q" und 3R-Benzyl-piperidin):
(R)-"R", amorph, Zers. bei 142°; [α] -24,0°.

**Beispiel 2**

Analog Beispiel 1 erhält man durch $NaBH_4$-Reduktion der nachstehenden Ketone

6-(2-(3-Benzyl-piperidino)-1-oxo-ethyl)-7-chlor-1,2,3,4-tetrahydro-chinolin-2-on

6-(2-(3-Benzyl-piperidino)-1-oxo-ethyl)-7-chlor-1,2,3,4-tetrahydro-4,4-dimethyl-chinolin-2-on

6-(2-(2-Benzyl-piperidino)-1-oxo-ethyl)-7-chlor-1,2,3,4-tetrahydro-4,4-dimethyl-chinolin-2-on

6-(2-(3-Benzyl-piperidino)-1-oxo-ethyl)-8-chlor-1,2,3,4-tetrahydro-4,4-dimethyl-chinolin-2-on

6-(2-(3-Benzyl-piperidino)-1-oxo-ethyl)-5-chlor-1,2,3,4-tetrahydro-4,4-dimethyl-chinolin-2-on

6-(2-(3-Benzyl-piperidino)-1-oxo-ethyl)-1,2-dihydro-4H-3,1-benzoxazin-2-on

6-(2-(3-Benzyl-piperidino)-1-oxo-ethyl)-1,2,3,4-tetrahydro-chinazolin-2-on

6-(2-(3-Benzyl-piperidino)-1-oxo-ethyl)-2,3-dihydro-benzothiazol-2-on

bzw. ihrer Enantiomeren die nachstehenden Verbindungen:

6-(2-(3-Benzyl-piperidino)-1-hydroxy-ethyl)-7-chlor-1,2,3,4-tetrahydro-chinolin-2-on

6-(2-(3S-Benzyl-piperidino)-1-hydroxy-ethyl)-7-chlor-1,2,3,4-tetrahydro-chinolin-2-on

6-(2-(3R-Benzyl-piperidino)-1-hydroxy-ethyl)-7-chlor-1,2,3,4-tetrahydro-chinolin-2-on

6-(2-(3-Benzyl-piperidino)-1-hydroxy-ethyl)-7-chlor-1,2,3,4-tetrahydro-4,4-dimethyl-chinolin-2-on

6-(2-(3S-Benzyl-piperidino)-1-hydroxy-ethyl)-7-chlor-1,2,3,4-tetrahydro-4,4-dimethyl-chinolin-2-on, Hydrochlorid, F. 221-222°; [α] +46,8°

6-(2-(3R-Benzyl-piperidino)-1-hydroxy-ethyl)-7-chlor-1,2,3,4-tetrahydro-4,4-dimethyl-chinolin-2-on, Hydrochlorid,

F. 201-203°; [α] -29,1°

6-(2-(2-Benzyl-piperidino)-1-hydroxy-ethyl)-7-chlor-1,2,3,4-tetrahydro-4,4-dimethyl-chinolin-2-on

6-(2-(2S*-Benzyl-piperidino)-1-hydroxy-ethyl)-7-chlor-1,2,3,4-tetrahydro-4,4-dimethyl-chinolin-2-on

6-(2-(2R*-Benzyl-piperidino)-1-hydroxy-ethyl)-7-chlor-1,2,3,4-tetrahydro-4,4-dimethyl-chinolin-2-on

6-(2-(3-Benzyl-piperidino)-1-hydroxy-ethyl)-8-chlor-1,2,3,4-tetrahydro-4,4-dimethyl-chinolin-2-on

6-(2-(3S-Benzyl-piperidino)-1-hydroxy-ethyl)-8-chlor-1,2,3,4-tetrahydro-4,4-dimethyl-chinolin-2-on

6-(2-(3R-Benzyl-piperidino)-1-hydroxy-ethyl)-8-chlor-1,2,3,4-tetrahydro-4,4-dimethyl-chinolin-2-on

6-(2-(3-Benzyl-piperidino)-1-hydroxy-ethyl)-5-chlor-1,2,3,4-tetrahydro-4,4-dimethyl-chinolin-2-on

6-(2-(3S-Benzyl-piperidino)-1-hydroxy-ethyl)-5-chlor-1,2,3,4-tetrahydro-4,4-dimethyl-chinolin-2-on

6-(2-(3R-Benzyl-piperidino)-1-hydroxy-ethyl)-5-chlor-1,2,3,4-tetrahydro-4,4-dimethyl-chinolin-2-on

6-(2-(3-Benzyl-piperidino)-1-hydroxy-ethyl)-1,2-dihydro-4H-3,1-benzoxazin-2-on

6-(2-(3S-Benzyl-piperidino)-1-hydroxy-ethyl)-1,2-dihydro-4H-3,1-benzoxazin-2-on

6-(2-(3R-Benzyl-piperidino)-1-hydroxy-ethyl)-1,2-dihydro-4H-3,1-benzoxazin-2-on

6-(2-(3-Benzyl-piperidino)-1-hydroxy-ethyl)-1,2,3,4-tetrahydro-chinazolin-2-on

6-(2-(3S-Benzyl-piperidino)-1-hydroxy-ethyl)-1,2,3,4-tetrahydro-chinazolin-2-on; F. 157-160°; [α] +29,7°;

6-(2-(3R-Benzyl-piperidino)-1-hydroxy-ethyl)-1,2,3,4-tetrahydro-chinazolin-2-on; F. 158-162°; [α] -27,9°;

6-(2-(3-Benzyl-piperidino)-1-hydroxy-ethyl)-2,3-dihydro-benzothiazol-2-on

6-(2-(3S-Benzyl-piperidino)-1-hydroxy-ethyl)-2,3-dihydro-benzothiazol-2-on

6-(2-(3R-Benzyl-piperidino)-1-hydroxy-ethyl)-2,3-dihydro-benzothiazol-2-on.

**Beispiel 3**

Eine Lösung von 7,1 g (+)-β-Chlor-diisopinocampheyl-boran in 20 ml Ether wird unter $N_2$ auf -70° gekühlt und unter Rühren tropfenweise mit einer Lösung von 1 g (S)-"A" in 15 ml THF versetzt. Man rührt noch 2 Std., läßt das Gemisch durch 16 Std. Stehen sich auf Raumtemperatur erwärmen und versetzt mit wässerig-methanolischer Salzsäure. Man trennt die Phasen, wäscht die wässerige Phase mit Hexan und macht mit NaOH alkalisch. Übliche Aufarbeitung liefert 6-(2-(3S-Benzyl-piperidino)-1R*-hydroxy-ethyl)-1,2,3,4-tetrahydro-chinolin-2-on, Hydrochlorid, F. 218-219° (Zers.).

Analog erhält man mit (-)-β-Chlor-diisopinocampheyl-boran aus (S)-"A" das 6-(2-(3S-Benzyl-piperidino)-1S*-hydroxy-ethyl)-1,2,3,4-tetrahydro-chinolin-2-on, Hydrochlorid, F. 221-223°.

**Beispiel 4**

Eine Lösung von 100 mg (S)-"A" in 10 ml Ethanol wird zugesetzt zu einer Kultur von Rhodutorula mucilaginosa in 1000 ml einer Nährlösung, die 1 % Hefeextrakt, 2 % Pepton aus Casein, 2 % Glucose und 0,1 % $KH_2PO_4$ enthält. Das Gemisch wird unter ständigem Schütteln 72 Std. bei 28° inkubiert. Man arbeitet wie üblich auf und erhält 6-(2-(3S-Benzyl-piperidino)-1S*-hydroxy-ethyl)-1,2,3,4-tetrahydro-chinolin-2-on, Hydrochlorid, F. 221-223°.

**Beispiel 5**

Ein Gemisch von 18,9 g 1,2,3,4-Tetrahydro-6-oxiranyl-chinolin-2-on (erhältlich durch Reduktion von "B" mit $NaBH_4$ zu 6-(2-Chlor-1-hydroxy-ethyl)-1,2,3,4-tetrahydro-chinolin-2-on und anschließende Behandlung mit Triethylamin in Ethanol bei 20°), 17,5 g 3R-Benzyl-piperidin, 15 g Triethylamin und 1000 ml Ethanol wird 2 Std. gekocht. Nach dem Abkühlen arbeitet man wie üblich auf und erhält 6-(2-(3R-Benzyl-piperidino)-1-hydroxy-ethyl)-1,2,3,4-tetrahydro-chinolin-2-on ((R)-"C"), F. 136-138°.

**Beispiel 6**

6-(3-Chlor-1-oxopropyl)-1,2,3,4-tetrahydrochinazolin-2-on

20 g Aluminiumchlorid (0,15 mol) werden in 100 ml Dichlormethan aufgenommen. Bei maximal 20 °C werden unter Rühren portionsweise 7,43 g 1,2,3,4-Tetrahydrochinazolin-2-on (0,05 mol) hinzugefügt. Das so erhaltene Reaktionsgemisch wird 30 Minuten nachgerührt. Anschließend wird unter Rühren bei einer Temperatur von maximal 25 °C eine Lösung, bestehend aus 6,98 g 3-Chlorpropionsäurechlorid (0,055 mol) und 50 ml Dichlormethan, zugetropft und noch eine Stunde nachgerührt. Nach Beendigung der Reaktion wird das erhaltene Reaktionsgemisch in 300 g Eis eingerührt, der Niederschlag abgesaugt und mit reichlich Wasser und geringen Mengen Methanol gewaschen. Durch die Reaktion wird ein geringfügig polareres Produkt als das Ausgangsmaterial gebildet, das sich dünnschichtchromatographisch mit einem Elutionsmittel, bestehend aus Chloroform und Methanol im Mischungsverhältnis 95:5, abtrennen läßt. Ausbeute: 11,37 g 6-(3-Chlor-1-oxopropyl-)-1,2,3,4-tetrahydro-chinazolin-2-on (95,3 % der Theorie); Fp: >270 °C

**Beispiel 7**

5-(3-Chlor-1-oxopropyl)-6-chlor-2,3-dihydrobenzimidazol-2-on

44,68 g Aluminiumchlorid (0,335 mol) werden in einen Reaktionskolben vorgelegt. Unter Rühren werden langsam 4,9 ml DMF zugetropft, wobei die Temperatur auf etwa 56 °C ansteigt. Zu diesem Gemisch werden 6,9 ml 3-Chlorpropionsäurechlorid (0,072 mol) hinzugefügt. Anschließend werden langsam portionsweise 8,07 g 6-Chlor-2,3-dihydrobenzimidazol-2-on (0,048 mol) hinzugefügt und eine Stunde bei 80 °C gerührt. Nach Beendigung der Reaktion wird das erhaltene Reaktionsgemisch in 400 g Eis eingerührt, der Niederschlag abgesaugt und mit reichlich Wasser und geringe Mengen Aceton gewaschen. Die Umsetzung führt zu einem geringfügig unpolareren Produkt, das sich dünnschichtchromatographisch mit einem Elutionsmittel, bestehend aus Chloroform und Methanol im Mischungsverhältnis 9:1, abtrennen läßt. Ausbeute: 9,72 g 5-(3-Chlor-1-oxopropyl)-6-chlor-2,3-dihydrobenzimidazol-2-on (78,2 % der Theorie); Fp: 201-204 °C

**Beispiel 8**

6-{3-[4-(4-Fluorbenzyl)-1-piperidyl]-1-oxopropyl}-1,2,3,4-tetrahydrochinazolin-2-on

2,39 g 6-(3-Chlor-1-oxopropyl-)-1,2,3,4-tetrahydrochinazolin-2-on (0,01 mol), 40 ml Acetonitril, 2,30 g 4-(4-Fluorbenzyl-)piperidin (0,01 mol) und 4,05 g Triethylamin (0,04 mol) werden zwei Stunden bei Raumtemperatur gerührt. Die Umsetzung führt zu einem geringfügig unpolareren Produkt als 4-(4-Fluorbenzyl)-piperidin, das sich dünnschichtchromatographisch mit einem Elutionsmittel, bestehend aus Chloroform und Methanol im Mischungsverhältnis 9:1, abtrennen läßt. Nach Beendigung der Reaktion wird das Reaktionsgemisch mit Wasser verdünnt, der Niederschlag abgesaugt und mit Aceton gewaschen. Anschließend wird das erhaltene Produkt chromatographisch an einer Kieselgelsäule gereinigt, wodurch das Umsetzungsprodukt in Form von farblosen Kristallen erhalten wird, die nochmals aus einem Methanol/Diethylether-Gemisch umkristallisiert werden. Ausbeute: 2,43 g 6-{3-[4-(4-Fluorbenzyl)-1-piperidyl]-1-oxopropyl}1,2,3,4-tetrahydrochinazolin-2-on (61,5 % der Theorie); Fp: 206-208 °C

Analog wurden weiterhin folgende Verbindungen hergestellt:

aus 5-(3-Chlor-1-oxopropyl)-2,3-dihydro-1H-benzimidazol-2-on und 4-(4-Fluorbenzyl)piperidin
5-{3-[4-(4-Fluorbenzyl)-piperidino]-1-oxopropyl}-2,3-dihydro-1H-benzimidazol-2-on, Smp. 187 - 190 °C

aus 5-(3-Chlor-1-oxopropyl)-2,3-dihydroindol-2-on und 4-(4-Fluorbenzyl)-piperidin
5-{3-[4-(4-Fluorbenzyl)-1-piperidyl]-1-oxopropyl}-2,3-dihydroindol-2-on, Smp. 172 - 173 °C

aus 7-Chlor-6-(3-chlor-1-oxopropyl)-1,2,3,4,tetrahydrochinolin-2-on und 4-(4-Fluorbenzyl)piperidin

6-(3-(4-(4-Fluorbenzyl)-1-piperidyl)-1-oxopropyl)-7-chlor-1,2,3,4-tetrahydrochinolin-2-on, Smp. 151 - 156 °C

aus 5-Fluor-6(-3-chlor-1-oxopropyl)-1H-2,3-dihydrobenzimidazol-2-on und 4-(4-Fluorbenzyl)piperidin
5-[3-[4-(4-Fluorbenzyl)-1-piperidyl]-1-oxopropyl]-6-fluor-2,3-dihydro-1H-benzimidazol-2-on, Smp. 197 - 199 °C

aus 6-(3-Chlor-1-oxopropyl)-3-methyl-2,3-dihydrobenzoxazol-2-on und 4-Benzylpiperidin
6-[3-(4-Benzyl-1-piperidyl)-1-oxopropyl]-2,3-dihydro-3-methylbenzoxazol-2-on, Smp. 131 - 132 °C

aus 7-Chlor-6-(3-chlor-1-oxopropyl)-1,2,3,4-tetrahydrochinolin-2-on und 4-Benzylpiperidin
6-(3-(4-Benzyl-1-piperidyl)-1-oxopropyl)-7-chlor-1,2,3,4-tetrahydro-chinolin-2-on, Smp. 146-147 °C

**Beispiel 9**

6-{3-(4-(4-Fluorbenzyl)-1-piperidyl-1-hydroxypropyl}-1,2,3,4-tetrahydrochinazol in-2-on

1,60 g 6-{3-[4-(4-Fluorbenzyl)-1-piperidyl]-1-oxopropyl}-1,2,3,4-tetrahydrochinazolin-2-on (0,00405 mol) werden in 20 ml Methanol suspendiert, unter Rühren und Kühlen mit einem Eis/Wasser-Gemisch werden portionsweise 0,15 g $NaBH_4$ (0,00405 mol) zugegeben.

Anschließend wird 16 Stunden bei Raumtemperatur nachgerührt. Zur vollständigen Umsetzung der Edukte wird anschließend nochmals $NaBH_4$ in kleinen Portionen zugegeben. Dann wird das Reaktionsgemisch mit etwa 50 ml Wasser verdünnt, der gebildete Niederschlag abgesaugt, mit Wasser gewaschen und aus einem Methanol/Wasser-Gemisch umkristallisiert. Ausbeute: 1,11 g 6-{3-[4-(4-Fluorbenzyl)-1-piperidyl]-1-hydroxypropyl}-1,2,3,4-tetrahydro-chinazolin-2-on (68,9 % der Theorie); Fp: 183- 185 °C

Analog wurden weiterhin folgende Verbindungen hergestellt:

aus 7-(3-(4-Benzyl-1-piperidyl)-1-oxopropyl)-3,4-dihydro-2H-1,4-benzoxazin-3-on und $NaBH_4$
7-(3-(4-Benzyl-1-piperidyl)-1-hydroxypropyl)-3,4-dihydro-2H-1,4-benzoxazin-3-on, Smp.:182-183 °C

aus (-)-5-{3-[(3R)-3-Benzylpipendino]-1-oxopropyl}-2,3-dihydro-1H-benzimidazol-2-on und $NaBH_4$
(-)-5-{3-[(3R)-3-Benzylpiperidino]-1-hydroxypropyl}-2,3-dihydro-1H-benzimidazol-2-on, Zersetzung ab 95 °C

aus (+)5-{3-[(3S)-3-Benzylpiperidino]-1-oxopropy}-2,3-dihydro-1H-benzimidazol-2-on und $NaBH_4$
(+)5-{3-[(3S)-3-Benzylpiperidino]-1-hydroxypropyl}-2,3-dihydro-1H-benzimidazol-2-on, Zersetzung ab 96 °C

aus 5-[3-(4-Benzylpiperidino)-1-oxopropyl]-2,3-dihydroindol-2-on und $NaBH_4$
5-[3-(4-Benzylpiperidino)-1-hydroxypropyl]-2,3-dihydroindol-2-on, Smp. 127-129 °C

aus (-)-5-{3-[(3R)-2-Benzylpiperidino]-1-oxopropyl}-2,3-dihydroindol-2-on und $NaBH_4$
(-)-5-{3-[(3R)-2-Benzylpiperidino]-1-hydroxypropyl}-2,3-dihydroindol-2-on, Smp. 160-164 °C

aus (+)-5-{3-[(3S)-3-Benzylpiperidino]-1-oxopropyl}-2,3-dihydroindol-2-on und $NaBH_4$
(+)-5-{3-[(3S)-3-Benzylpiperidino]-1-hydroxypropyl}-2,3-dihydroindol-2-on, Smp. 160-164 °C

aus 6-[3-(4-Benzyl-1-piperidinyl)-1-oxopropyl]-1,2,3-4-tetrahydrochinazolin-2-on und $NaBH_4$
6-[3-(4-Benzyl-1-piperidinyl)-1-hydroxypropyl]-1,2,3-4-tetrahydro-chinazolin-2-on, Smp. 146-149 °C

aus (-)-6-{3-[(3R)-3-Benzyl-1-piperidyl]-1-oxopropyl}-1,2,3,4-tetrahydrochinazolin-2-on und $NaBH_4$
(-)-6-{3-[(3R)-3-Benzyl-1-piperidyl]-1-hydroxypropyl}-1,2,3,4-tetrahydrochinazolin-2-on, Smp. 130 - 133°C

aus 6-[3-(4-Benzyl-1-piperidinyl)-1-oxopropyl]-8-chlor-4,4-dimethyl-1,2,3,4-tetrahydrochinolin-2-on und $NaBH_4$
6-[3-(4-Benzyl-1-piperidinyl)-1-hydroxypropyl]-8-chlor-4,4-dimethyl-1,2,3,4-tetrahydrochinolin-2-on, Smp. 93 - 95 °C

aus (+)-6-{3-[(3S)-2-Benzyl-1-piperidyl]-1-oxopropyl}-1,2,3,4-tetrahydrochinazolin-2-on und $NaBH_4$
(+)-6-{3-[(3S)-2-Benzyl-1-piperidyl]-1-hydroxypropyl}-1,2,3,4-tetrahydrochinazolin-2-on, Smp. 128-130 °C

aus 6-{3-[(3R)-3-[Benzyl-1-piperidyl]-1-oxopropyl}-8-chlor-4,4-dimethyl-1,2,3,4-tetrahydrochinolin-2-on und $NaBH_4$
6-{3-[(3R)-3-Benzyl-1-piperidyl]-1-hydroxypropyl}-8-chlor-4,4-dimethyl-1,2,3,4-tetrahydrochinolin-2-on, Harz

aus 6-{3-[(3S)-3-Benzyl-1-piperidyl]-1-oxopropyl}-8-chlor-4,4-dimethyl-1,2,3,4-tetrahydrochinolin-2-on und $NaBH_4$
6-{3-[(3S)-3-Benzyl-1-piperidyl]-1-hydroxpropyl}-8-chlor-4,4-dimethyl-1,2,3,4-tetrahydrochinolin-2-on, Harz

aus 7-(3-((3S)-3-Benzyl-1-piperidyl)-1-oxopropyl)-3,4-dihydro-2H-1,4-benzoxazin-3-on und $NaBH_4$
7-(3-((3S)-3-Benzyl-1-piperidyl)-1-hydroxypropyl)-3,4-dihydro-2H-1,4-benzoxazin-3-on, Smp. 128-132 °C

aus 7-(3-((3R)-3-Benzyl-1-piperidyl)-1-oxopropyl)-3,4-dihydro-2H-1,4-benzoxazin-3-on und $NaBH_4$
7-(3-((3R)-3-Benzyl-1-piperidyl)-1-hydroxypropyl)-3,4-dihydro-2H-1,4-benzoxazin-3-on, Smp. 132-140 °C

aus 6-[3-(4-Benzylpiperidino)-1-oxopropyl]-5-chlor-2,3-dihydro-benzoxazol-2-on und $NaBH_4$
6-[3-(4-Benzylpiperidino)-1-hydroxypropyl]-5-chlor-2,3-dihydro-benzoxazol-2-on, Zersetzung ab 94 °C

aus (-)-6-{3-[(3R)-3-Benzylpiperidino]-1-oxopropyl}-5-chlor-2,3-dihydrobenzoxazol-2-on und $NaBH_4$
(-)-6-{3-[(3R)-3-Benzylpiperidino]-1-hydroxypropyl}-5-chlor-2,3-dihydrobenzoxazol-2-on, Zersetzung ab 98 °C

aus (+)-6-{3-[(3S)-3-Benzylpiperidino]-1-oxopropyl}-5-chlor-2,3-dihydro-benzoxazol-2-on und $NaBH_4$
(+)-6-{3-[(3S)-3-Benzylpiperidino]-1-hydroxypropyl}-5-chlor-2,3-dihydrobenzoxazol-2-on, Zersetzung ab 98 °C

aus 6-[3-(4-Benzyl-1-piperidyl)-1-oxopropyl]-1,2,3,4-tetrahydrochinolin-2-on und $NaBH_4$
6-[3-(4-Benzyl-1-piperidyl)-1-hydroxypropyl]-1,2,3,4-tetrahydro-chinolin-2-on, Smp. 137-138 °C

aus 6-(3-(4-Benzyl-1-pipendinyl)-1-oxopropyl)-7-chlor-1,2,3,4-tetrahydrochinolin-2-on und $NaBH_4$
6-(3-(4-Benzyl-1-piperidinyl)-1-hydroxypropyl)-7-chlor-1,2,3,4-tetrahydrochinolin-2-on, Smp. 181-184 °C

aus (-)-6-{3-[(3R)-3-Benzyl-1-piperidyl]-1-oxopropyl}-1,2,3,4-tetrahydrochinolin-2-on und $NaBH_4$
(-)-6-{3-[(3R)-3-Benzyl-1-pipendyl]-1-hydroxypropyl}-1,2,3,4-tetrahydrochinolin-2-on, Smp. 182 °C

aus 6-{3-[(3S)-3-Benzyl-1-piperidyl]-1-oxopropyl}-1,2,3,4-tetrahydrochinolin-2-on und $NaBH_4$
6-{3-[(3S)-3-Benzyl-1-piperidyl]-1-hydroxypropyl}-1,2,3,4-tetrahydrochinolin-2-on, Smp. 182-185 °C

aus (+)-6-((3S)-3-Benzyl-1-piperidinyl)-1-oxopropyl)-7-chlor-1,2,3,4-tetrahydrochinolin-2-on und $NaBH_4$
(+)-6-((3S)-3-Benzyl-1-piperidinyl)-1-hydroxypropyl)-7-chlor-1,2,3,4-tetrahydrochinolin-2-on, Smp. 123-125°C

aus (-)-6-((3R)-3-Benzyl-1-piperidinyl-)-1-oxopropyl)-7-chlor-1,2,3,4-tetrahydrochinolin-2-on und $NaBH_4$
(-)-6-((3R)-3-Benzyl-1-piperidinyl-)-1-hydroxypropyl)-7-chlor-1,2,3,4-tetrahydrochinolin-2-on, Smp. 122-125 °C

aus 6-(3-(4-Benzyl-1-piperidyl)-1-oxoethyl)-5-chlor-1,2,3,4-tetrahydrochinolin-2-on und $NaBH_4$
6-(3-(4-Benzyl-1-piperidinyl)-1-hydroxyethyl)-5-chlor-1,2,3,4-tetrahydrochinolin-2-on, Smp. 224-227 °C

aus 5-{3-[4-(4-Fluorbenzyl)-piperidino]-1-oxopropyl}-2,3-dihydro-1H-benzimidazol-2-on und $NaBH_4$
5-{3-[4-(4-Fluorbenzyl)-piperidino]-1-hydroxypropyl}-2,3-dihydro-1H-benzimidazol-2-on, Smp. 218-221 °C

aus 5-[3-(4-Benzyl-1-pipendyl)-1-oxopropyl]-6-chlor-2,3-dihydro-1H-benzimidazol-2-on und $NaBH_4$
5-[3-(4-Benzyl-1-piperidyl)-1-hydroxypropyl]-6-chlor-2,3-dihydro-1H-benzimidazol-2-on, Smp. 233-236 °C

aus (-)-5-{[3-(3R)-3-Benzyl-1-piperidyl]-1-oxopropyl}-6-chlor-2,3-dihydro-1H-benzimidazol-2-on und $NaBH_4$
(-)-5-{[3-(3R)-3-Benzyl-1-piperidyl]-1-hydroxypropyl}-6-chlor-2,3-dihydro-1H-benzimidazol-2-on, Zersetzung ab 115 °C

aus (+)-5-{3-[(3S)-3-Benzyl-1-piperidyl]-1-oxopropyl}-6-chlor-2,3-dihydro-1H-benzimidazol-2-on und $NaBH_4$
(+)-5-{3-[(3S)-3-Benzyl-1-piperidyl]-1-hydroxypropyl}-6-chlor-2,3-dihydro-1H-benzimidazol-2-on, Zersetzung ab 94 °C

aus 6-Chlor-5-{3-[4-(4-fluorbenzyl)-1-piperidyl]-1-oxopropyl}-2,3-dihydro-1H-benzimidazol-2-on und $NaBH_4$
6-Chlor-5-{3-[4-(4-fluorbenzyl)-1-piperidyl]-1-hydroxypropyl}-2,3-dihydro-1H-benzimidazol-2-on, Smp. 246-248 °C

aus 6-[3-(4-Benzyl-1-piperidyl)-1-oxopropyl]-2,3-dihydrobenzoxazol-2-on und $NaBH_4$

6-[3-(4-benzyl-1-piperidyl)-1-hydroxypropyl]-2,3-dihydrobenzoxazol-2-on, Smp. 177-178°C

aus 5-{3-[4-(4-Fluorbenzyl)-1-piperidyl]-1-oxopropyl}-2,3-dihydroindol-2-on und $NaBH_4$
5-{3-[4-(4-Fluorbenzyl)-1-piperidyl]-1-hydroxypropyl}-2,3-dihydroindol-2-on, Smp. 154-155 °C

aus 7-[3-(4-Benzyl-1-piperidyl)-1-oxopropyl]-2,3,4,5-tetrahydro-1H-1-benzazepin-2-on und $NaBH_4$
7-[3-(4-Benzyl-1-piperidyl)-1-hydroxypropyl]-2,3,4,5-tetrahydro-1H-1-benzazepin-2-on, Smp. 140-141 °C

aus 6-(3-(4-Fluorbenzyl-1-piperidyl)-1-oxopropyl)-7-chlor-1,2,3,4-tetrahydrochinolin-2-on und $NaBH_4$
6-(3-(4-Fluorbenzyl-1-piperidyl)-1-hydroxypropyl)-7-chlor-1,2,3,4-tetrahydrochinolin-2-on, Smp. 220-222 °C

aus 7-{3-[(3R)-3-Benzyl-1-piperidyl]-1-oxopropyl}-2,3,4,5-tetrahydro-1H-1-benzazepin-2-on und $NaBH_4$
7-{3-[(3R)-3-Benzyl-1-piperidyl]-1-hydroxypropyl}-2,3,4,5-tetrahydro-1H-1-benzazepin-2-on, Harz

aus (-)-6-{2-[(3S)-3-Benzylpiperidino]-1-oxoethyl}-5-chlor-2,3-dihydro-benzoxazol-2-on und $NaBH_4$
(-)-6-{2-[(3S)-3-Benzylpiperidino]-1-hydroxyethyl}-5-chlor-2,3-dihydro-benzoxazol-2-on-Hydrochlorid, Zersetzung ab 147 °C

aus (+)-6-{2-[(3R)-3-Benzylpiperidino]-1-oxoethyl}-5-chlor-2,3-dihydro-benzoxazol-2-on und $NaBH_4$
(+)-6-(2-[(3R)-3-Benzylpiperidino]-1-hydroxyethyl}-5-chlor-2,3-dihydro-benzoxazol-2-on-Hydrochlorid, Zersetzung ab 144 °C

aus (+)-6-{2-[(3S)-3-Benzyl-1-piperidyl)-1-oxoethyl]-7-chlor-1,2,3,4-tetrahydrochinolin-2-on und $NaBH_4$
(+)-6-(2-((3S)-3-Benzyl-1-piperidyl)-1-hydroxyethyl)-7-chlor-1,2,3,4-tetrahydrochinolin-2-on, Zersetzung bei 107-117 °C

aus (-)-6-(2-((3R)-3-Benzyl-1-piperidyl)-1-oxoethyl)-7-chlor-1,2,3,4-tetrahydrochinolin-2-on und $NaBH_4$
(-)-6-(2-((3R)-3-Benzyl-1-piperidyl)-1-hydroxyethyl)-7-chlor-1,2,3,4-tetrahydrochinolin-2-on, amorph

aus 7-[2-(4-Benzyl-1-piperidyl)-1-oxoethyl]-2,3,4,5-tetrahydro-1H-1-benzazepin-2-on und $NaBH_4$
7-[2-(4-Benzyl-1-piperidyl)-1-hydroxyethyl]-2,3,4,5-tetrahydro-1H-1-benzazepin-2-on-Hydrochlorid, Zersetzung bei 238-239 °C

aus (-)-7-{2-[(3R)-3-Benzyl-1-piperidyl]-1-oxoethyl}-2,3,4,5-tetrahydro-1H-1-benzazepin-2-on und $NaBH_4$
(-)-7-{2-[(3R)-3-Benzyl-1-piperidyl]-1-hydroxyethyl}-2,3,4,5-tetrahydro-1H-1-benzazepin-2-on-Dihydrochlorid-hydrat, Smp. 115-118 °C

aus (+)-7-{2-[(3S)-3-Benzyl-1-piperidyl]-1-oxoethyl}-2,3,4,5-tetrahydro-1H-1-benzazepin-2-on und $NaBH_4$
(+)-7-{2-[(3S)-3-Benzyl-1-piperidyl]-1-hydroxyethyl}-2,3,4,5-tetrahydro-1H-1-benzazepin-2-on-Dihydrochlorid-Hydrat, Smp. 115-118 °C

aus (+)-6-(2-((3S)-3-Benzyl-1-piperidyl)-1-oxoethyl)-5-chlor-1,2,3,4-tetrahydrochinolin-2-on und $NaBH_4$
(+)-6-(2-((3S)-3-Benzyl-1-piperidyl)-1-hdroxyethyl)-5-chlor-1,2,3,4-tetrahydrochinolin-2-on, Smp. 119-120 °C

aus (-)-6-(2-((3R)-3-Benzyl-1-piperidyl)-1-oxoethyl)-5-chlor-1,2,3,4-tetrahydrochinolin-2-on und $NaBH_4$
(-)-6-(2-((3R)-3-Benzyl-1-piperidyl)-1-hydroxyethyl)-5-chlor-1,2,3,4-tetrahydrochinolin-2-on, Smp. 119-125 °C

aus 5-[3-(4-Benzyl-1-piperidyl)-1-oxopropyl]-6-fluor-2,3-dihydro-1H-benzimidazol-2-on und $NaBH_4$
5-[3-(4-Benzyl-1-piperidyl)-1-hydroxypropyl]-6-fluor-2,3-dihydro-1H-benzimidazol-2-on, Smp. 244-247 °C

aus (-)-5-{3-[(3R)-3-Benzyl-1-piperidy]-1-oxopropyl}-6-fluor-2,3-dihydro-1H-benzimidazol-2-on und $NaBH_4$
(-)-5-{3-[(3R)-3-Benzyl-1 -piperidyl]-1-hydroxypropyl}-6-fluor-2,3-dihydro-1H-benzimidazol-2-on, $[\alpha]_D^{20}$ = -15,1° (DMSO)

aus (+)-5-{3-[(3S)-3-Benzyl-1-piperidyl]-1-oxopropyl}-6-fluor-2,3-dihydro-1H-benzimidazol-2-on und $NaBH_4$
(+)-5-{3-[(3S)-3-Benzyl-1-piperidyl]-1-hxdroxypropyl}-6-fluor-2,3-dihydro-1H-benzimidazol-2-on, $[\alpha]_D^{20}$ = -15,8° (DMSO)

aus 5-{3-[4-(4-Fluorbenzyl)-1-piperidyl]-1-oxopropyl}-6-fluor-2,3-dihydro-1H-benzimidazol-2-on und $NaBH_4$
5-{3-[4-(4-Fluorbenzyl)-1-piperidyl]-1-hydroxypropyl}-6-fluor-2,3-dihydro-1H-benzimidazol-2-on, Smp. 202-205 °C

**Beispiel 10**

5-[3-(4-Benzyl-1-piperidy)-1-oxopropyl]-2,3-dihydrobenzimidazol-2-on

a) 2,25 g (0,01 mol) des analog Beispiel 6 hergestellten 5-(3-Chloro-1-oxopropyl)-2,3-dihydrobenzimidazol-2-ons werden in 40 ml Acetonitril aufgenommen. Zu diesem Gemisch werden unter Rühren 1,75 g 4-Benzylpiperidin (0,01 mol) und 3,04 g Triethylamin (0,03 mol) hinzugefügt. Das erhaltene Reaktionsgemisch wird zwei Stunden bei Raumtemperatur gerührt. Anschließend wird die Reaktionslösung mit 100 ml Dichlormethan verdünnt und mit 70 ml Wasser geschüttelt. Die Phasen werden getrennt. Nach dem Trocknen der organischen Phase wird unter Vakuum das Lösungsmittel abdestilliert.
Ausbeute: 3,64 g 5-[3-(4-Benzyl-1-piperidyl)-1-oxopropyl)-2,3-dihydrobenzimidazol-2-on

b) Die Reaktion wird wie in a) durchgeführt, jedoch wird der in der Reaktion entstandene Niederschlag abgesaugt und mehrmals mit Wasser gewaschen. Anschließend wird das Rohprodukt in 200 ml Dichlormethan und 10 ml Methanol gelöst. Die Lösung wird filtriert und getrocknet. Nach dem Trocknen wird unter Vakuum das Lösungsmittelgemisch abdestilliert. Der so erhaltene Rückstand wird chromatographisch an einer Kieselgelsäule mit einem Dichlormethan/Methanol-Gemisch als Elutionsmittel aufgetrennt, wobei neben dem Umsetzungsprodukt unpolare Verunreinigungen erhalten werden. Das in Form von gelben Kristallen erhaltene Reaktionsprodukt wird in Aceton aufgekocht und umkristallisiert.
Ausbeute: 1,52 g 5-[3-(4-Benzyl-1-piperidyl)-1-oxopropyl]-2,3-dihydrobenzimidazol-2-on (41,9% der Theorie); Fp: 183-186 °C

Analog wurden weiterhin folgende Verbindungen hergestellt:

aus 6-(3-Chlor-1-oxopropyl)-2,3-dihydrobenzoxazol-2-on und 4-Benzylpiperidin
6-[3-(4-Benzyl-1-piperidyl)-1-oxopropyl]-2,3-dihydrobenzoxazol-2-on, Smp. 161-162 °C

aus 7-(3-Chlor-1-oxopropyl)-2,3,4,5-tetrahydro-1H-1-benzazepin-2-on und 4-Benzylpiperidin
7-[3-(4-Benzyl-1-piperidyl)-1-oxopropyl]-2,3,4,5-tetrahydro-1H-1-benzazepin-2-on, Harz

aus 7-(3-Chlor-1-oxopropyl)-2,3,4,5-tetrahydro-1H-1-benzazepin-2-on und 3-R-Benzylpiperidin
7-{3-[(3R)-3-Benzyl-1-piperidyl]-1-oxopropyl}-2,3,4,5-tetrahydro-1H-1-benzazepin-2-on, Harz

aus 6-Fluor-5-(3-chlor-1-oxopropyl)-2,3-dihydro-1H-benzimidazol-2-on und 4-Benzylpiperidin
5-[3-(4-Benzyl-1-piperidyl)-1-oxopropyl]-6-fluor-2,3-dihydro-1H-benzimidazol-2-on, Smp. 205-206 °C

aus 5-(3-Chlor-1-oxopropyl)-2,3-dihydro-1-methyl-benzimidazol-2-on und 4-Benzylpiperidin
5-[3-(4-Benzyl-1-piperidinyl)-1-oxopropyl]-2,3-dihydro-1-methylbenzimidazol-2-on, Smp. 154-157 °C

aus 6-Fluor-5-(3-chlor-1-oxopropyl)-1H-2,3-dihyrobenzimidazol-2-on und 3-R-Benzylpiperidin
(-)-5-{3-[(3R)-3-Benzyl-1-piperidyl]-1-oxopropyl}-6-fluor-2,3-dihydro-1H-benzimidazol-2-on, Smp. 193-194 °C

aus 6-Fluor-5-(3-chlor-1-oxopropyl)-1H-2,3-dihyrobenzimidazol-2-on und 3-S-Benzylpiperidin
(+)-5-{3-[(3S)-3-Benzyl-1-piperidyl]-1-oxopropyl}-6-fluor-2,3-dihydro-1H-benzimidazol-2-on, Smp. 192-194 °C

aus 6-(3-Chlor-1-oxopropyl)-1,2,3,4-tetrahydrochinazolin-2-on und 4-Benzylpiperidin
6-[3-(4-Benzyl-1-piperidyl)-1-oxopropyl]-1,2,3,4-tetrahydro-chinazolin-2-on, Smp 196-199 °C

aus 6-(3-Chlor-1-oxopropyl)-2,3-dihydrobenzoxazol und 4-(4-Fluorbenzyl)piperidin
6-{3-[4-(4-Fluorbenzyl)-1-piperidyl]-1-oxopropyl}-2,3-dihydrobenzoxazol-2-on, Smp. 168-173 °C

**Beispiel 11**

5-[3-(4-Benzyl-1-piperidyl)-1-hydroxypropyl]-2,3-dihydro-2-oxo-benzimidazol

3,64 g (0,01 mol) des Reaktionsprodukts aus Beispiel 10 werden in 40 ml Methanol suspendiert. Zu dieser Suspension werden unter Rühren und Kühlung mit einer Eis/Wasser-Mischung portionsweise 0,38 g $NaBH_4$ (0,01 mol) gegeben. Anschließend wird bei Raumtemperatur eine Stunde nachgerührt. Das erhaltene Reaktionsgemisch wird mit 60 ml Wasser verdünnt und noch 30 Minuten gerührt. Der gebildete Niederschlag wird abgesaugt und mit 50 ml Methanol gekocht bis sich feine hellgelbe Kristalle gebildet haben. Die Kristalle werden abgesaugt und mit Diethylether nachgewaschen. Ausbeute: 2,34 g 5-[3-(4-Benzyl-1-piperidyl)-1-hydroxypropyl]-2,3-dihydro-2-oxo-benzimidazol (64,1 % der Theorie); Fp: 240-244 °C
Die nachstehenden Beispiele betreffen pharmazeutische Zubereitungen.

**Beispiel A: Injektionsgläser**

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4$ x 2 $H_2O$, 28,48 g $Na_2HPO_4$ x 12 $H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann beispielsweise in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

1. Benzylpiperidinderivate der Formel 1

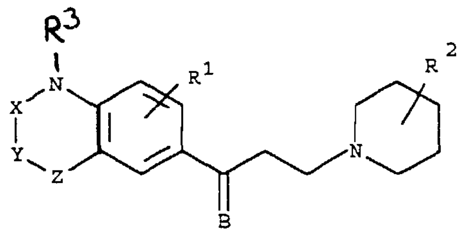

(1)

worin

$R^1$ H, Hal oder Nitro
$R^2$ eine unsubstituierte oder durch Hal am Aromaten substituierte Benzylgruppe in 2-,3- oder 4-Stellung des Piperidinrings, mit der Maßgabe, daß $R^2 \neq$ 4-Benzyl, wenn X-CO-, Y und Z $-CH_2$ und $R^1$H bedeuten,
$R^3$ H oder A
X -CO- oder $-SO_2-$,
Y $-CH_2-$, -NH-,-O-, -S- oder auch -CO-, wenn X-CO- und Z-NH-, -NA-,
Z $-CH_2-$, $-C(A)_2-$, $-CH_2-CH_2-$, -CH=CH-, -CO-,-NH-,-NA-,-O- oder eine Bindung,

wobei einer der Reste X, Y und Z -O-, -S-, oder -NH- sein kann, jedoch X-Y bzw. Y-Z nicht -O-O-,-S-S-,-NH-O-, -O-NH-, -NH-NH-, -O-S-, -S-O- ist,

A Alkyl mit 1-6 C-Atomen

und

B O,H +OH,
Hal F, Cl, Br und I

bedeuten, sowie deren Salze.

**2.** a) 1,2,3,4-Tetrahydro-6-(1-hydroxy-2,3-benzyl-piperidino)-ethyl)-chinolin-2-on, dessen Diastereomere und Enantiomere sowie die Salze dieser Verbindungen.

b) 5-[3-(4-Benzylpiperidino)-1-hydroxypropyl]-2,3-dihydro-2-oxo-1H-benzimidazol

c) 5-{3-[4-(4-Fluorbenzyl)-piperidino]-1-oxopropyl}-2,3-dihydro-1H-benzimidazol-2-on

**3.** Verfahren zur Herstellung von Benzylpiperidinderivaten der Formel 1 nach Anspruch 1 sowie von deren Salzen, dadurch gekennzeichnet, daß eine Verbindung der Formel (II)

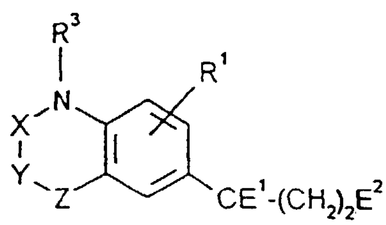

(II)

worin

$E^1$ O oder H + OH,

$E^2$ Cl, Br, J oder eine reaktionsfähig veresterte OH-Gruppe bedeuten oder

$E^1$ und $E^2$ zusammen auch ein O-Atom bedeuten können und $R^1$, $R^3$, X, Y und

Z die angegebenen Bedeutungen haben,

mit einer Verbindung der Formel III

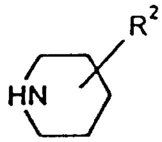

(III)

worin

$R^2$ die angegebene Bedeutung hat,

umsetzt,
und/oder
daß man eine sonst der Formel I entsprechende Verbindung, die jedoch an Stelle eines oder mehrerer H-Atome eine oder mehrere reduzierbare Gruppen und/oder eine oder mehrere zusätzliche Bindungen enthält, mit einem reduzierenden Mittel behandelt,
und/oder daß man eine erhaltene Base der Formel I durch Behandeln mit einer Säure in eines ihrer Säureadditionssalze umwandelt.

**4.** Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestes einem festen, flüssigen oder halbflüssigen Träger- und/oder Hilfsstoff in eine geeignete Dosierungsform bringt.

**5.** Pharmazeutische Zubereitung, gekennzeichnet durch einen Gehalt an mindestens einer Verbindung der allgemeine Formel I gemäß Anspruch 1 und/oder einem ihrer physiologisch unbedenklichen Salze.

**6.** Verwendung von Verbindungen der Formel I nach Anspruch 1 oder von deren physiologisch unbedenklichen Salzen zur Herstellung eines Arzneimittels.

**7.** Verwendung von Verbindungen der Formel I nach Anspruch 1 oder von deren physiologisch unbedenklichen Salzen zur Herstellung von Arzneimitteln zur Behandlung der Ischämie.

## Claims

**1.** Benzylpiperidine derivatives of the formula I

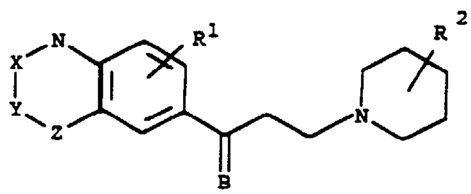

(I)

in which

$R^1$ is H, Hal or nitro,
$R^2$ is a benzyl group, which is unsubstituted or substituted by Hal on the aromatic, in, the 2-, 3- or 4-position of the piperidine ring, with the proviso that $R^2 \neq$ 4-benzyl if X is -CO-, Y and Z are $-CH_2-$ and $R^X$ is H,
$R^3$ is H or A,
X is -CO- or $-SO_2-$,
Y is $-CH_2-$, -NH-, -O-, -S- or alternatively -CO- if X is -CO- and Z is -NH- or -NA-,

Z is $-CH_2-$, $-C(A)_2-$, $-CH_2CH_2-$, -CH=CH-, -CO-, -NH-, -NA-, -O- or a bond,

where one of the radicals X, Y and Z can be -O-, -S- or -NH-, but X-Y or Y-Z is not -O-O-, -S-S-, -NH-O-, -O-NH-, -NH-NH-, -O-S- or -S-O-,

A is alkyl having 1 - 6 C atoms,
B is O, H + OH and
Hal is F, Cl, Br or I

and their salts.

**2.** a) 1,2,3,4-Tetrahydro-6-(1-hydroxy-2-(3-benzylpiperidino)ethyl)quinolin-2-one, its diastereomers and enantiomers, and the salts of these compounds.

b) 5-[3-(4-Benzylpiperidino)-1-hydroxypropyl]-2,3-dihydro-2-oxo-1H-benzimidazole

c) 5-{3-[4-(4-Fluorobenzyl)piperidino]-1-oxo-propyl}-2,3-dihydro-1H-benzimidazol-2-one

**3.** Process for the preparation of benzylpiperidine derivatives of the formula I according to Claim 1, and of their salts, characterized in that a compound of the formula (II)

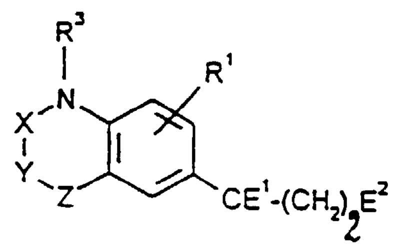

(II)

in which

$E^1$ is O or H + OH,

$E^2$ is Cl, Br, I or a reactive esterified OH group or

$E^1$ and $E^2$ together can be an O atom and
$R^1$, $R^3$, X, Y and Z have the meanings indicated,

is reacted with a compound of the formula III

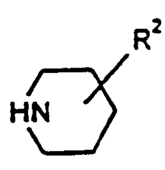

(III)

in which

$R^2$ has the meaning indicated,

and/or
in that a compound which otherwise corresponds to the formula I, but which instead of one or more H atoms contains one or more reducible groups and/or one or more additional bonds, is treated with a reducing agent, and/or in that a base of the formula I which is obtained is converted into one of its acid addition salts by treating with an acid.

4. Process for the production of pharmaceutical preparations, characterized in that a compound of the formula I according to Claim 1 and/or one of its physiologically acceptable salts is brought into a suitable dose form together with at least one solid, liquid or semi-liquid excipient and/or auxiliary.

5. Pharmaceutical preparation, characterized in that it contains at least one compound of the general formula I according to Claim 1 and/or one of its physiologically acceptable salts.

6. Use of compounds of the formula I according to Claim 1 or of their physiologically acceptable salts for the production of a medicament.

7. Use of compounds of the formula I according to Claim 1 or of their physiologically acceptable salts for the production of medicaments for the treatment of ischaemia.

**Revendications**

1. Dérivés de benzylpipéridine de formule I

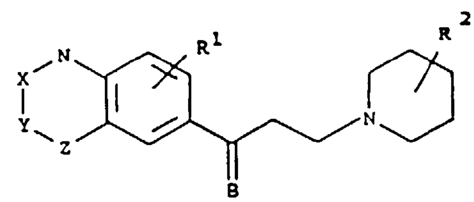

(I)

dans laquelle

$R^1$ représente un atome d'hydrogène ou d'halogéne ou le groupe nitro,
$R^2$ représente un groupe benzyle en position 2, 3 ou 4 du cycle pipéridine, non substitué ou substitué par un atome d'halogène sur le noyau aromatique, étant entendu que $R^2$ n'est pas le groupe 4-benzyle lorsque X représente -CO-, Y et Z représentent $-CH_2-$ et $R^1$ est H,
$R^3$ représente H ou A,
X représente -CO- ou $-SO_2-$,
Y représente $-CH_2-$, -NH-, -O-, -S- ou bien -CO- lorsque X représente -CO- et Z représente -NH-, -NA-,
Z représente $CH_2$, $-C(A)_2-$, $-CH_2-CH_2-$, -CH=CH-, -CO-, -NH-, -NA-, -O- ou une liaison,

l'un des radicaux X, Y et Z pouvant être -O-, -S- ou -NH-, mais X-Y ou Y-Z n'étant pas -O-O-, -S-S-, -NH-O-, -O-NH-, -NH-NH-, -O-S-, -S-O-,

A représente un groupe alkyle ayant de 1 à 6 atomes de carbone

et

B représente O, H + OH,
Hal représente F, Cl, Br et I

ainsi que leurs sels.

2. a) 1,2,3,4-tétrahydro-6-(1-hydroxy-2-(3-benzyl-pipéridino)éthyl)quinolin-2-one, ses diastéréoisomères et énantiomères, et sels de ces composés;
b) 5-[3-(4-benzylpipéridino)-1-hydroxypropyl]-2,3-dihydro-2-oxo-lH-benzimidazole,
c) 5-{3-[4-(4-fluorobenzyl)pipéridino]-1-oxo-propyl}-2,3-dihydro-1H-benzimidazole-2-one.

3. Procédé pour la préparation des dérivés de benzylpipéridine de formule I selon la revendication 1 ainsi que de leurs sels, caractérisé en ce que l'on fait réagir un composé de formule (II)

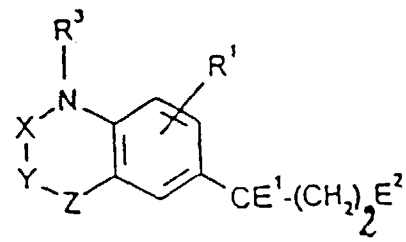

(II)

dans laquelle

| | |
|---|---|
| $E^1$ | représente O ou H + OH, |
| $E^2$ | représente Cl, Br, I ou un groupe OH estérifié réactif, ou |
| $E^1$ et $E^2$ | peuvent également représenter ensemble un atome d'oxygène, et |
| $R^1$, $R^3$, X, Y et Z | ont les significations indiquées, |

avec un composé de formule III

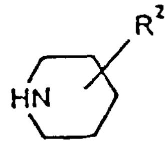

(III)

dans laquelle

$R^2$ a la signification indiquée,

et/ou
en ce que l'on traite par un réducteur un composé, correspondant par ailleurs à la formule I, mais qui contient au lieu d'un ou de plusieurs atomes d'hydrogène un ou plusieurs groupes réductibles et/ou une ou plusieurs liaisons supplémentaires,
et/ou
en ce que l'on convertit une base obtenue de formule I en un de ses sels d'addition avec des acides, par traitement par un acide.

**4.** Procédé pour la préparation de compositions pharmaceutiques, caractérisé en ce que l'on met sous une forme galénique appropriée un composé de formule I selon la revendication 1 et/ou un de ses sels physiologiquement acceptables, conjointement avec au moins un adjuvant et/ou véhicule solide, liquide ou semi-liquide.

**5.** Composition pharmaceutique, caractérisée par une teneur en au moins un composé de formule générale I selon la revendication 1, et/ou en un de ses sels physiologiquement acceptables.

**6.** Utilisation des composés de formule I selon la revendication 1 ou de leurs sels physiologiquement acceptables, pour la fabrication d'un médicament.

**7.** Utilisation des composés de formule I selon la revendication 1 ou de leurs sels physiologiquement acceptables, pour la fabrication de médicaments destinés au traitement de l'ischémie.